# EUROPEAN PATENT APPLICATION

(11) **EP 2 732 767 A1**
(43) Date of publication of application: **21.05.2014**
(21) Application number: 13190562.2
(22) Date of filing: 29.10.2013
(51) Int. Cl.: A61B 8/00, F16M 11/42, B60B 33/02

(54) **Ultrasonic diagnostic apparatus**

(30) Priority: 19.11.2012 KR 20120130804
(71) Applicant: Samsung Medison Co., Ltd., Gangwon-do 250-870 (KR)
(72) Inventor: Kim, Seung Tae, Seoul (KR)
(74) Representative: Lorenz, Markus

(57) **Abstract**

An ultrasonic diagnostic apparatus includes a main body, multiple caster modules configured to enable the main body to move, and a power transmission device. Each of the caster modules includes a wheel bracket, a wheel attached to the wheel bracket to be in contact with a ground, and a lever laterally and rotatably mounted to a top of the wheel bracket in order to change modes of the caster modules. The power transmission device transmits external force to the caster modules in order to change the modes of the caster modules, and includes a connection bar connecting levers to each other and configured to move laterally by the external force, a cam member having a cam surface and configured to rotate by the external force, a rolling member configured to move along the cam surface, and an elastic member configured to elastically bias the rolling member toward the cam surface.

## Description

### TECHNICAL FIELD

The present inventive concept relates to an ultrasonic diagnostic apparatus equipped with caster modules for movement thereof, and a pedal to change modes of the caster modules.

### BACKGROUND

In general, ultrasonic diagnostic apparatuses direct ultrasonic signals from a body surface of an object to a desired region inside a human body, and obtain an image related to a mono layer of soft tissue or a blood-stream using the ultrasonic signals reflected from the desired region, i.e., obtain information of the ultrasonic echo signals in a non-invasive manner.

An ultrasonic diagnostic apparatus includes a main body, a probe to transmit an ultrasonic signal to an object to be examined and receive an echo signal reflected from the object, a display unit disposed above the main body in order to display an ultrasonic image corresponding to a diagnostic result acquired from the received ultrasonic signal, and a control panel disposed in front of the display unit in order to enable a user to manipulate the ultrasonic diagnostic apparatus.

Such an ultrasonic diagnostic apparatus further includes caster modules mounted to a bottom of the main body in order to enable a user to easily move the ultrasonic diagnostic apparatus, and a pedal to change modes of the caster modules by being pressed by a user.

### SUMMARY

An aspect of the present inventive concept relates to an ultrasonic diagnostic apparatus equipped with a pedal assembly in order to change modes of caster modules.

Additional aspects of the inventive concept will be set forth in part in the description which follows and, in part, will be obvious from the description, or may be learned by practice of the inventive concept.

One aspect of the present inventive concept encompasses an ultrasonic diagnostic apparatus including a main body, a plurality of caster modules configured to enable the main body to move, and a power transmission device configured to transmit external force to the caster modules in order to change the modes of the caster modules. Each of the plural caster modules includes a wheel bracket, a wheel attached to the wheel bracket to be in contact with a ground, and a lever laterally and rotatably mounted to a top of the wheel bracket in order to change modes of the caster modules. The power transmission device includes a connection bar connecting the levers to each other and configured to move laterally by the external force, a cam member having a cam surface and configured to rotate by the external force, a rolling member configured to move along the cam surface, and an elastic member configured to elastically bias the rolling member toward the cam surface.

The ultrasonic diagnostic apparatus may further include a pedal configured to rotate vertically by external force exerted thereon by a user. The power transmission device may further include an interlocking link having a first end connected to the pedal and a second end rotatably mounted to the connection bar and configured to rotate laterally corresponding to the vertical rotation of the pedal, and a ball joint having a first end connected to the pedal and a second end connected to the interlocking link. The connection bar may move laterally corresponding to the lateral rotation of the interlocking link. The cam member may be disposed at the interlocking link and may rotate laterally with the interlocking link.

The ultrasonic diagnostic apparatus may further include a rotating bar having a first end and a second end mounted with the rolling member, the rotating bar being configured to rotate about the first end thereof. The elastic member may elastically bias the second end of the rotating bar to rotate toward the cam surface.

The elastic member may include a torsion spring mounted to the first end of the rotating bar.

The levers may rotate laterally to any one of an aligned position in which the wheels are aligned in one direction, a locked position in which positions of the wheels are locked, and an unlocked position in which the wheels are free to rotate laterally.

The cam surface may include a first concave surface guiding movement of the levers between the locked position and the unlocked position, a convex surface formed adjacent to the first concave surface to guide movement of the levers between the unlocked position and the aligned position, and a second concave surface disposed adjacent to the convex surface.

The ultrasonic diagnostic apparatus may further include a pedal bracket mounted to the main body, and a pedal cover shielding the pedal. A rear end portion of the pedal is rotatably mounted to the pedal bracket,

As described above, the ultrasonic diagnostic apparatus enables a user to more easily change the modes of the caster modules by operation of the power transmission device including the cam member and the rolling member elastically biased toward the cam surface of the cam member.

Also, a user may more clearly recognize the mode change of the caster modules due to the cam member and the rolling member.

### BRIEF DESCRIPTION OF THE DRAWINGS

The foregoing and other features of the inventive concept will be apparent from more particular description of embodiments of the inventive concept, as illustrated in the accompanying drawings in which like reference characters may refer to the same or similar parts throughout the different views. The drawings are not necessarily to scale, emphasis instead being placed upon illustrating the principles of the embodiments of the inventive concept. In the drawings, the thickness of layers and regions may be exaggerated for clarity.
FIG. 1 is a perspective view showing an ultrasonic diagnostic apparatus according to an embodiment of the present inventive concept.
FIG. 2 is an exploded perspective view showing caster modules, a pedal assembly and a power transmission device of an ultrasonic diagnostic apparatus according to an embodiment of the present inventive concept.
FIG. 3 is an exploded perspective view showing a pedal assembly and a power transmission device of an ultrasonic diagnostic apparatus according to an embodiment of the present inventive concept;
FIGS. 4 through 6 are views schematically showing operation of a pedal assembly and a power transmission device of an ultrasonic diagnostic apparatus according to an embodiment of the present inventive concept.
FIG. 7 is a view schematically showing movement of a rolling member along a cam surface.

### DETAILED DESCRIPTION

Examples of the present inventive concept will be described below in more detail with reference to the accompanying drawings. The examples of the present inventive concept may, however, be embodied in different forms and should not be construed as limited to the examples set forth herein. Like reference numerals may refer to like elements throughout the specification.

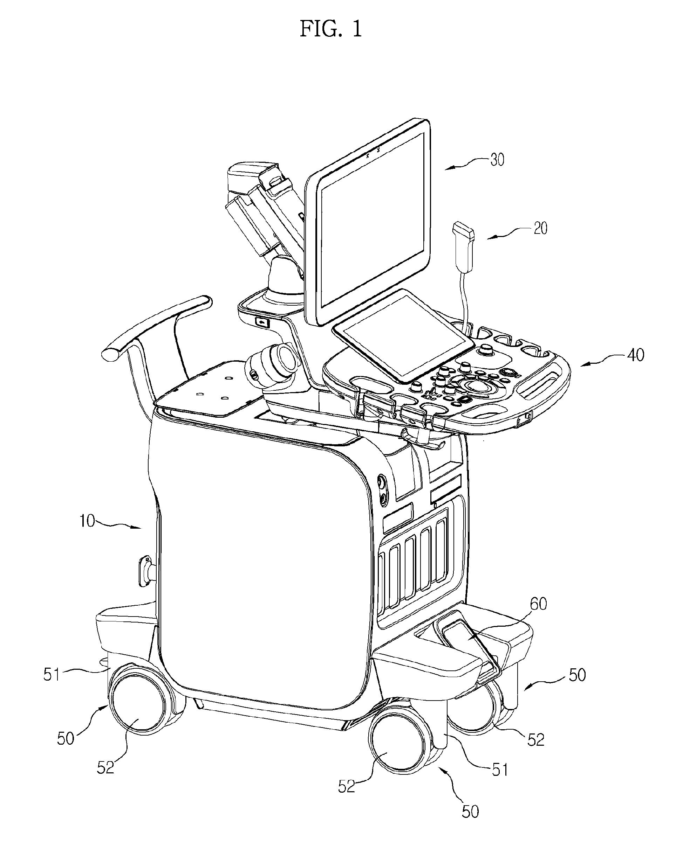

As shown in FIG. 1, an ultrasonic diagnostic apparatus according to an embodiment of the present inventive concept may includes a main body 10, a probe 20 to transmit an ultrasonic signal to an object to be examined and receive an echo signal reflected from the object, a display unit 30 disposed above the main body 10 in order to display an ultrasonic image corresponding to a diagnostic result acquired from the received ultrasonic signal, and a control panel 40 to enable a user to manipulate the ultrasonic diagnostic apparatus.

The ultrasonic diagnostic apparatus may further include plural caster modules 50 mounted to a bottom of the main body 10 so that a user may move the ultrasonic diagnostic apparatus. In an embodiment of the present inventive concept, the main body 10 may have an approximately hexahedral shape, and thus four caster modules 50 may be provided.

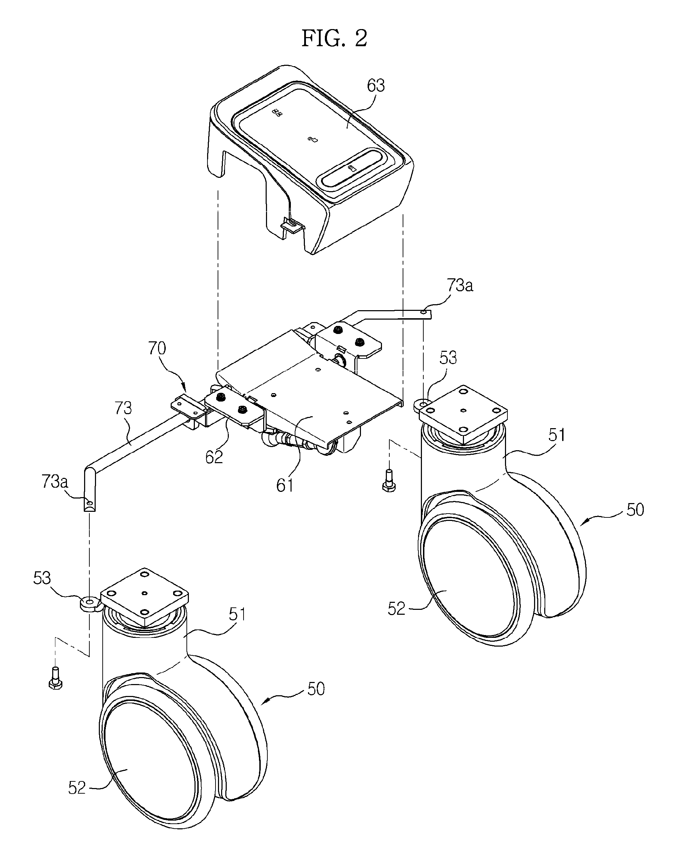

As shown in FIGS. 1 and 2, each of the caster modules 50 may include a wheel bracket 51 and a wheel 52 attached to the wheel bracket 51 so as to be in contact with the ground.

Such caster modules 50 may be constructed to selectively perform an aligned mode in which the wheels 52 are aligned in one direction, a locked mode in which the wheels 52 are locked at a certain position, or an unlocked mode in which the wheels 52 are free to rotate laterally. So as to selectively perform these modes, levers 53 may be laterally and rotatably mounted to tops of the wheel brackets 51.

While rotating laterally, the levers 53 may be located at any one of a locked position to perform the locked mode by locking the wheels 52 at a certain position, an aligned position to perform the aligned mode by aligning the wheels 52 in one direction, and an unlocked position to perform the unlocked mode by allowing the wheels 52 to freely rotate laterally.

In order to rotate the levers 53, the ultrasonic diagnostic apparatus further includes a pedal assembly 60 configured to rotate vertically by being pressed by a user, and a power transmission device 70 to transmit external force applied to the pedal assembly 60 to the levers 53.

Referring to FIG. 2, the pedal assembly 60 (see FIG. 1) may include a pedal 61 which rotates vertically by external force applied by a user, a pedal bracket 62 to which the pedal 61 is vertically and rotatably mounted, and a pedal cover 63 to shield the pedal 61.

The pedal 61 may be vertically and rotatably mounted to the pedal bracket 62 using a hinge shaft 61a to connect a rear end portion of the pedal 61 to the pedal bracket 62.

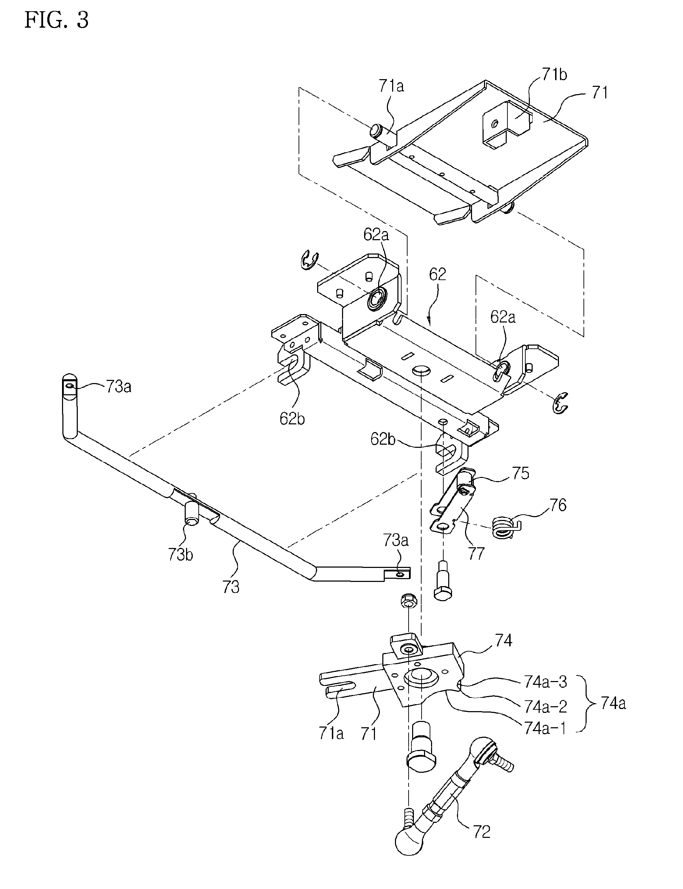

As shown in FIG. 3, the pedal bracket 62 may include a pair of hinge holes 62a through which both ends of the hinge shaft 61a are rotatably inserted. A pair of support slots 62b through which a connection bar 73 (which will be described later) may be laterally and movably inserted.

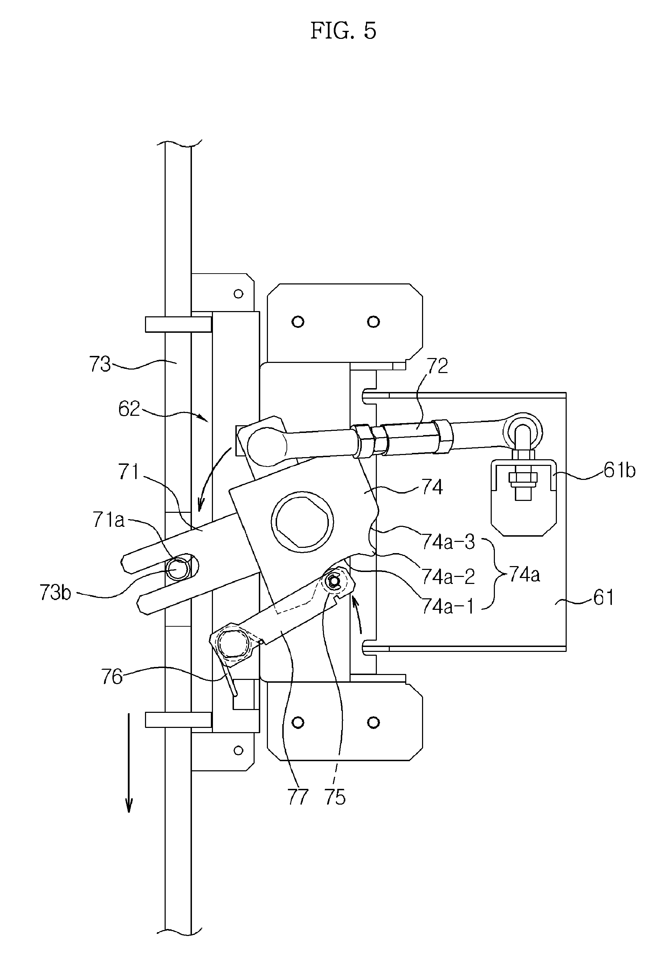

The power transmission device 70, as shown in FIGS. 2 and 3, may include an interlocking link 71 which is laterally and rotatably mounted to the pedal bracket 62. The interlocking link 71 may rotate laterally corresponding to vertical rotation of the pedal 61. The power transmission device 70 may also include a ball joint 72 (see FIGS. 3 and 5) which has one end connected to the pedal 61 and the other end connected to the interlocking link 71 in order to transmit external force to the interlocking link 71, and the connection bar 73 which connects the levers 53 of the caster modules 50 to each other. An end of the interlocking link 71 may be rotatably coupled to the connection bar 73. Accordingly, when the interlocking link 71 rotates laterally, the connection bar 73 may move laterally, to thereby rotate the levers 53 laterally.

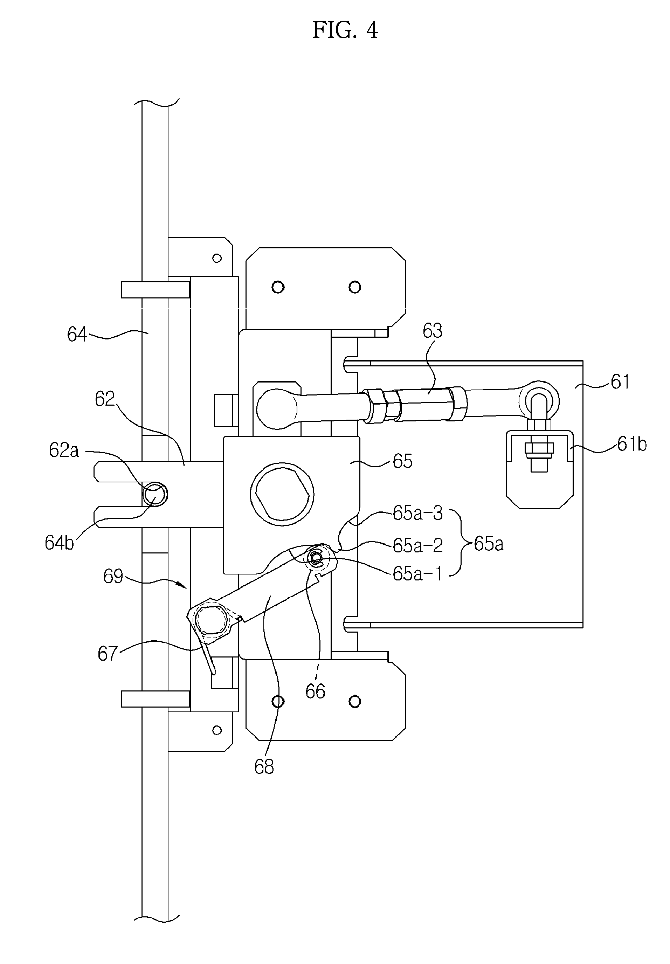

The ball joint 72 may have an end coupled to a joint bracket 61b (see FIGS. 4 and 5). The joint bracket 61b may be mounted to a bottom surface of the pedal 61.

The interlocking link 71 may have an approximately L-shape, and may be laterally and rotatably mounted to the pedal bracket 62. The interlocking link 71 may include one end formed with a guide slot 71a to guide lateral movement of the connection bar 73, and the other end coupled with the other end of the ball joint 72.

The connection bar 73 may include a pair of connection parts 73a respectively formed at both ends thereof in order to be respectively coupled with the levers 53, and a guide protrusion 73b provided at the middle portion thereof and inserted through the guide slot 71a of the interlocking link 71 so as to receive force from the interlocking link 71.

If a user presses a pedal 61 and thus the pedal 61 rotates vertically about the hinge shaft 61a, the vertical rotational force of the pedal 61 may be transmitted to the interlocking link 71 through the ball joint 72, and accordingly the interlocking link 71 may rotate laterally. Consequently, the connection bar 73 may move laterally, and the positions of the levers 53 of the respective caster modules 50 may be simultaneously changed corresponding to the lateral movement of the connection bar 73. As a result, all of the caster modules 50 may operate in the same mode.

The force necessary to change the positions of the levers 53 of the caster modules 50 may not be uniform. In detail, a relatively large force may be necessary to move the levers 53 from the unlocked position to the locked position, however, such large force may not be necessary to move the levers 53 from the locked position to the unlocked position.

Further, when moving the levers 53 from the unlocked position to the aligned position or from the aligned position to the unlocked position, a relatively small force may be necessary. Therefore, a user may hardly feel the position change of the levers 53.

The power transmission device 70 may further include a cam member 74 formed with a cam surface 74a at a portion thereof, a rolling member 75 configured to move along the cam surface 74a, and an elastic member 76 configured to elastically bias the rolling member 75 toward the cam surface 74a so as to be in contact with the cam surface 74a. Due to the power transmission device 70, a force necessary to change the positions of the levers 53 may be different on a case-by-case basis.

The power transmission device 70 may further include a rotating bar 77 (see FIGS. 3 and 5) which has one end rotatably coupled to the pedal bracket 62 and the other end mounted with the rolling member 75. The elastic member 76 is located at the end of the rotating bar 77, and thus the rotating bar 77 may rotate in one direction by an elastic restoring force of the elastic member 76, thereby elastically biasing the rolling member 75 located at the other end of the rotating bar 77 toward the cam surface 74a. In an embodiment of the present inventive concept, the elastic member 76 may be configured as a torsion spring.

The cam surface 74a may include a first concave surface 74a-1 to guide movement of the levers 53 between the locked position and the unlocked position, a convex surface 74a-2 formed adjacent to the first concave surface 74a-1 to guide movement of the levers 53 between the unlocked position and the aligned position, and a second concave surface 74a-3 formed adjacent to the convex surface 74a-2.

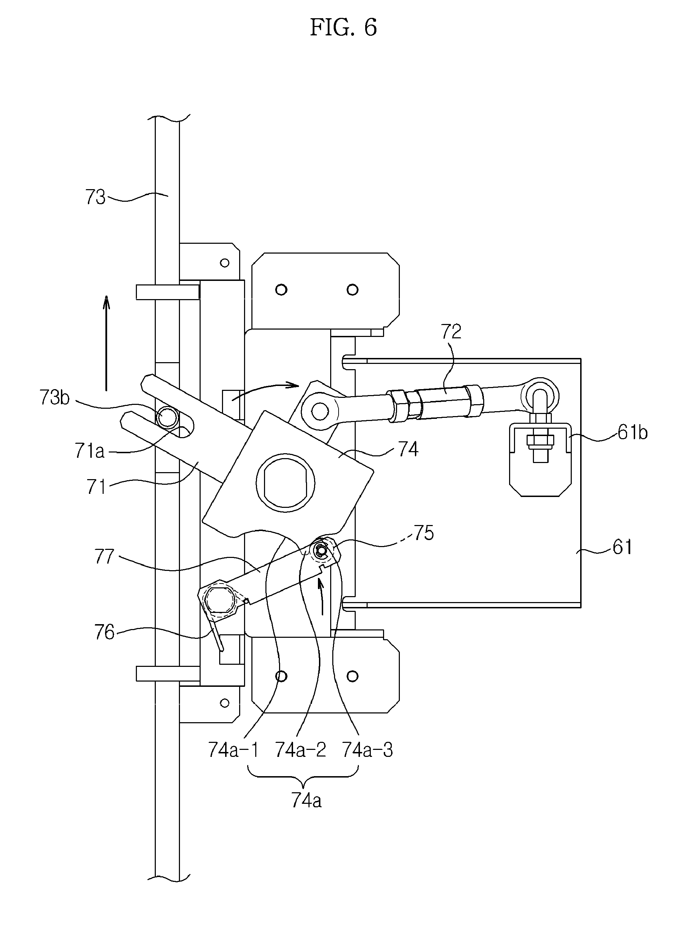

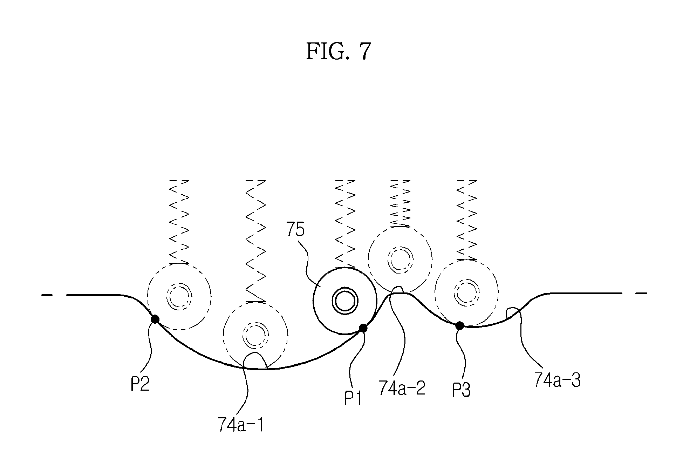

FIG. 4 shows a position of the rolling member 75 when the levers are in the unlocked position. FIG. 5 shows a position of the rolling member 75 when the levers are in the locked position. FIG. 6 shows a position of the rolling member 75 when the levers are in the aligned position. FIG. 7 shows movement of the rolling member 75 along the cam surface 74a.

As illustrated in the drawings, when the rolling member 75 moves between the unlocked position P1 (see FIG. 7) and the locked position P2 (see FIG. 7), the elastic member 76 may be elastically restored. Such an elastic restoring force of the elastic member 76 may be applied in the same direction as external force exerted on the pedal 61 by a user. Therefore, although the force exerted on the pedal 61 by a user is as small as the elastic restoring force of the elastic member 76, the levers 53 may move from the unlocked position P1 to the locked position P2. Also when the rolling member 75 moves from the locked position P2 to the unlocked position P1, the elastic restoring force of the elastic member 76 may contribute to the rotation of the levers 53. In other words, a force necessary to move the levers 53 from the locked position to the unlocked position or from the unlocked position to the locked position may be reduced.

In order to move the rolling member 75 from the unlocked position P1 to the aligned position P3 (see FIG. 7) or from the aligned position P3 to the unlocked position P1, the rolling member 75 may be caused to pass along the convex surface 74a-2 of the cam surface 74a. At this time, the elastic member 76, which elastically biases the rotating bar 77, may be additionally elastically deformed. Accordingly, in order to move the levers 53 from the unlocked position to the aligned position, an additional force to elastically deform the elastic member 76 may be necessarily exerted on the pedal 61.

Since a user is required to apply a certain amount of force to the pedal 61 so that the rolling member 75 may pass along the convex surface 74a-2, the user may recognize that the levers 53 are in either the unlocked or aligned position.

Although a few embodiments of the present inventive concept have been shown and described, it would be appreciated by those skilled in the art that changes may be made in these embodiments without departing from the principles and spirit of the inventive concept, the scope of which is defined in the claims and their equivalents.

## Claims

1. An ultrasonic diagnostic apparatus, comprising:
a main body;
a plurality of caster modules configured to enable the main body to move, each of the plurality of caster modules including:
a wheel bracket,
a wheel attached to the wheel bracket to be in contact with a ground, and
a lever laterally and rotatably mounted to a top of the wheel bracket in order to change modes of the caster modules; and
a power transmission device configured to transmit external force to the caster modules in order to change the modes of the caster modules, the power transmission device including:
a connection bar connecting levers of the caster modules to each other and configured to move laterally by the external force,
a cam member having a cam surface and configured to rotate by the external force,
a rolling member configured to move along the cam surface, and
an elastic member configured to elastically bias the rolling member toward the cam surface.

2. The ultrasonic diagnostic apparatus according to claim 1, further comprising:
a pedal configured to rotate vertically by external force exerted thereon by a user, wherein:
the power transmission device further includes:
an interlocking link having a first end connected to the pedal and a second end rotatably mounted to the connection bar and configured to rotate laterally corresponding to the vertical rotation of the pedal, and
a ball joint having a first end connected to the pedal and a second end connected to the interlocking link,
the connection bar moves laterally corresponding to the lateral rotation of the interlocking link, and
the cam member is disposed at the interlocking link and rotates laterally with the interlocking link.

3. The ultrasonic diagnostic apparatus according to claim 1, further comprising:
a rotating bar having a first end and a second end mounted with the rolling member, the rotating bar being configured to rotate about the first end thereof,
wherein the elastic member elastically biases the second end of the rotating bar to rotate toward the cam surface.

4. The ultrasonic diagnostic apparatus according to claim 3, wherein the elastic member includes a torsion spring mounted to the first end of the rotating bar.

5. The ultrasonic diagnostic apparatus according to claim 1, wherein the levers rotate laterally to any one of an aligned position in which the wheels are aligned in one direction, a locked position in which positions of the wheels are locked, and an unlocked position in which the wheels are free to rotate laterally.

6. The ultrasonic diagnostic apparatus according to claim 5, wherein the cam surface includes:
a first concave surface guiding movement of the levers between the locked position and the unlocked position,
a convex surface disposed adjacent to the first concave surface to guide movement of the levers between the unlocked position and the aligned position, and
a second concave surface disposed adjacent to the convex surface.

7. The ultrasonic diagnostic apparatus according to claim 2, further comprising:
a pedal bracket mounted to the main body, a rear end portion of the pedal being rotatably mounted to the pedal braket; and
a pedal cover shielding the pedal.

8. An ultrasonic diagnostic apparatus, comprising:
a plurality of caster modules, each including:
a wheel bracket,
a wheel attached to the wheel bracket to be in contact with a ground, and
a lever laterally and rotatably mounted to a top of the wheel bracket in
order to change modes of the caster modules;
a pedal configured to rotate vertically by external force exerted thereon by a user;
and
a power transmission device configured to transmit external force to the caster modules in order to change the modes of the caster modules, the power transmission device including:
a connection bar connecting levers of the caster modules to each other and configured to move laterally by the external force,
an interlocking link having a first end connected to the pedal and a second end rotatably mounted to the connection bar and configured to rotate laterally corresponding to the vertical rotation of the pedal, and
a ball joint having a first end connected to the pedal and a second end connected to the interlocking link.

9. The ultrasonic diagnostic apparatus according to claim 8, wherein the power transmission device further includes:
a cam member having a cam surface and configured to rotate by the external force,
a rolling member configured to move along the cam surface,
an elastic member configured to elastically bias the rolling member toward the cam surface.

10. The ultrasonic diagnostic apparatus according to claim 8, wherein the connection bar moves laterally corresponding to the lateral rotation of the interlocking link.

11. The ultrasonic diagnostic apparatus according to claim 9, wherein the cam member is disposed at the interlocking link and rotates laterally with the interlocking link.
